# EUROPÄISCHE PATENTSCHRIFT

(11) **EP 0 988 290 B1**
(45) Veröffentlichungstag und Bekanntmachung des Hinweises auf die Patenterteilung: **10.01.2007**
(21) Anmeldenummer: 98932150.0
(22) Anmeldetag: 10.06.1998
(51) Int. Cl.: C07D 233/60

(54) **CARBONYLDIIMIDAZOLE, DARAUS ABGELEITETE ESTER UND VERFAHREN ZU IHRER HERSTELLUNG**
CARBONYLDIIMIDAZOLES, THEIR ESTER DERIVATIVES AND METHOD FOR THEIR PRODUCTION
CARBONYLDIIMIDAZOLES, ESTERS DERIVES DE CES DERNIERS ET PROCEDE DE PREPARATION CORRESPONDANT

(30) Priorität: 12.06.1997 DE 19724884
(43) Veröffentlichungstag der Anmeldung: 29.03.2000
(73) Patentinhaber: BASF Aktiengesellschaft, 67056 Ludwigshafen (DE)
(72) Erfinder: STAMM, Armin, D-55128 Mainz (DE); JULIUS, Manfred, D-67117 Limburgerhof (DE); KINDLER, Alois, D-67454 Ha loch (DE); HENNINGSEN, Michael, D-67227 Frankenthal (DE); BOTZEM, Jörg, D-67117 Limburgerhof (DE)
(74) Vertreter: Isenbruck, Günter
(86) Internationale Anmeldenummer: PCT/EP1998/003516
(87) Internationale Veröffentlichungsnummer: WO 1998/056769

(56) Entgegenhaltungen:
- EP-A- 0 692 476
- W. JOHN: "Über einige Imidazol-carbonsäure-ester" CHEM. BER., Bd. 68, 1935, Seiten 2283-2291, XP002077052
- H. J. J. LOOZEN ET AL.: "Thermal Decarboxylation of N-Alkoxycarbonylimidazoles. An Improved and Convenient Procedure for N-Alkylation of Imidazoles" J. ORG. CHEM., Bd. 40, 1975, Seiten 3279-3280, XP002077053
- S. B. DAMLE, C.-Y. CHOU: "t-Butyloxycarbonyl Blocked Amino Acids for Peptide Synthesis" SPEC. CHEM., Bd. 13, 1993, Seiten 67-69, XP002077054 in der Anmeldung erwähnt
- W. KLEE, M. BRENNER: "t-Butyloxycarbonyl-imidazol und t-Butyloxycarbonylhydrazin" HELV. CHIM. ACTA, Bd. XLIV, Nr. VII, 1961, Seiten 2151-2153, XP002077055 in der Anmeldung erwähnt

## Beschreibung

Die Erfindung betrifft bestimmte Carbonyldiimidazole, daraus abgeleitete Ester, Verfahren zu deren Herstellung und deren Verwendung.

In der Peptidsynthese aus Aminosäuren ist der Schutz der an der Reaktion nicht beteiligten funktionellen Gruppen zwingend erforderlich, um ein einheitliches Produkt zu erhalten. Eine der bedeutendsten auch industriell verwendeten Schutzgruppen für die Aminogruppe von Aminosäuren ist die tert.-Butyloxycarbonyl-Schutzgruppe, die in der Regel als BOC abgekürzt wird. Die Aminofunktion wird dabei als Urethan geschützt, wobei die Schutzgruppe unter milden sauren Bedingungen wieder abspaltbar ist.

Normalerweise werden Urethane durch Umsetzung von Aminogruppen mit einem Chlorameisensäureester hergestellt. Der Chlorameisensäure-tert.-butylester ist jedoch außerordentlich instabil und kann daher nur schlecht gehandhabt werden. Er ist zur Einführung der BOC-Schutzgruppe daher ungeeignet. In der Literatur sind zahlreiche Verbindungen beschrieben, mit deren Hilfe Aminogruppen mit BOC-Schutzgruppen geschützt werden sollen.

Die am häufigsten zur Einführung von BOC-Schutzgruppen verwendete Verbindung ist Di-tert.-butyldicarbonat, auch als Pyrokohlensäuredi-tert.-butylester oder BOC-Anhydrid bezeichnet ((CH₃)₃C-O-CO-O-CO-O-C(CH₃)₃. Verfahren zur Herstellung von BOC-Anhydrid sind beispielsweise aus
US 5,162,565 und EP-A-0 468 404 bekannt. Die Herstellungsverfahren für BOC-Anhydrid sind mehrstufig und beinhalten aufwendige Verfahrensschritte unter Einsatz kostspieliger Chemikalien. Daher ist BOC-Anhydrid sehr teuer und wird ungern als Verbindung in industriellen Reaktionen eingesetzt.

Ein Beispiel anderer Verbindungen zur Einführung der BOC-Schutzgruppe ist BOC-Fluorid, wie es in Liebigs Ann. Chem. 716 (1968), 175 bis 185 beschrieben ist.

In J. Org. Chem. 50 (1985), 3951 bis 3953 ist die Herstellung von 1,2,2,2-Tetrachlor-ethyl-tert.-butylcarbonat und seine Verwendung zur Einführung von BOC-Schutzgruppen beschrieben.

In Helv. Chim. Acta 44 (1961), 2151, Spec. Chem. 13 (1993), 67 bis 69 und EP-A-0 236 888 ist die Herstellung von tert.-Butyloxycarbonylimidazol aus Carbonyldiimidazol und seine Verwendung zur Einführung der BOC-Schutzgruppe beschrieben.

Während BOC-Fluorid nur über das aufwendig herstellbare Carbonylfluoridchlorid synthetisiert werden kann und daher nur eine geringe Bedeutung hat, handelt es sich bei den beiden anderen Verbindungen um Feststoffe, die aufwendige Verfahrenstechniken zur Isolierung und Reinigung erfordern. Weitere BOC-Einführungsreagenzien haben wegen ihrer Instabilität keine technische Bedeutung.

Aufgabe der vorliegenden Erfindung ist die Bereitstellung von Verbindungen zur Einführung von BOC-Schutzgruppen, die aus einfach zugänglichen Verbindungen über wenig aufwendige Verfahrensschritte zugänglich sind. Die Verbindungen sollen ferner bei Raumtemperatur flüssig und bei vermindertem Druck unzersetzt destillierbar sein. Sie sollen ferner eine hohe Lagerstabilität besitzen und die Nachteile der bekannten Verbindungen vermeiden.

Die Aufgabe wird erfindungsgemäß gelöst durch Imidazolcarbonsäureester der allgemeinen Formeln IIIa und IIIb oder Gemische davon

Druck unzersetzt destillierbar sein. Sie sollen ferner eine hohe Lagerstabilität besitzen und die Nachteile der bekannten Verbindungen vermeiden.

Die Aufgabe wird erfindungsgemäß gelöst durch Imidazolcarbonsäureester der allgemeinen Formeln IIIa und IIIb oder Gemische davon 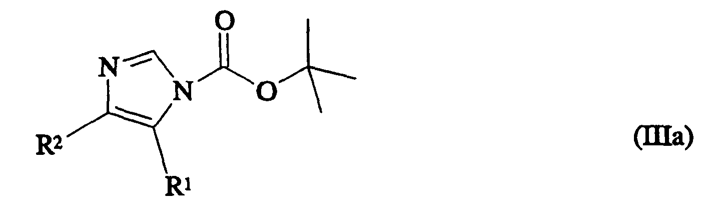 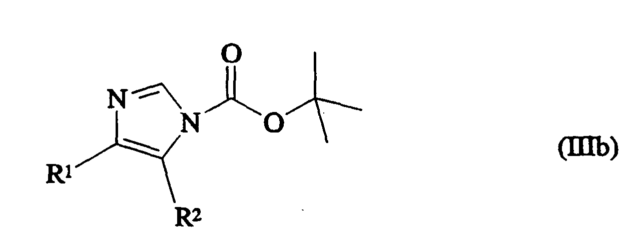 wobei R¹ ein C₁₋₄-Alkylrest und R² Wasserstoff oder ein Methylrest ist.

Die Imidazolcarbonsäureester der allgemeinen Formeln IIIa, IIIb oder Gemische davon können durch Umsetzung von Carbonyldiimidazolen der allgemeinen Formeln Ia, Ib, Ic oder Gemischen davon 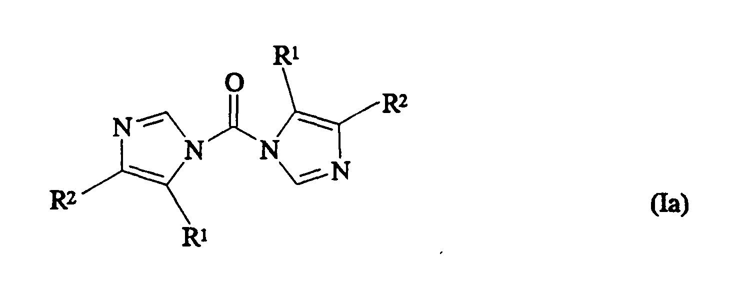 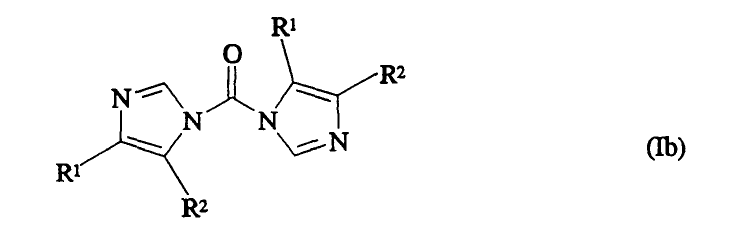 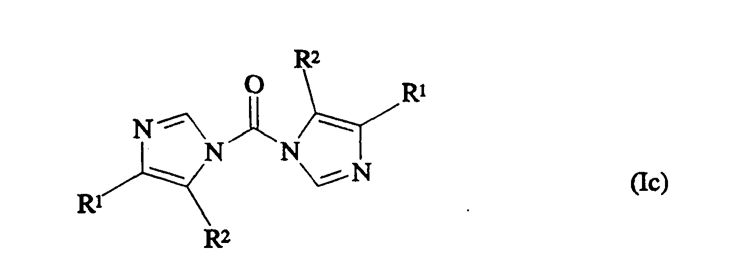 wobei R¹ ein C₁₋₄-Alkylrest und R² Wasserstoff oder ein Methylrest ist,
mit tert.-Butanol in einem substituierten aromatischen Kohlenwasserstoff als Lösungsmittel und Extrahieren des erhaltenen Reaktionsgemisches mit Wasser zur Entfernung von weiterhin gebildeten Imidazolen der allgemeinen Formeln IIa und IIb 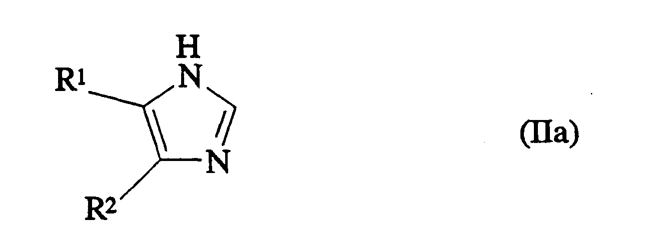 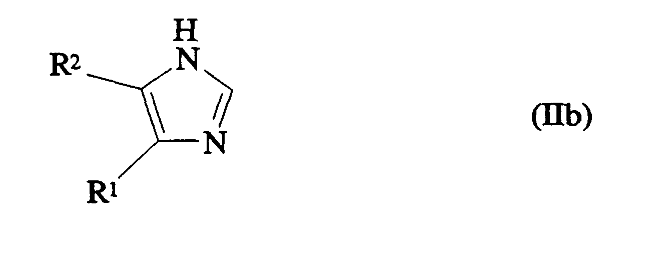 in denen R¹ und R² die vorstehende Bedeutung haben, hergestellt werden.

Die Carbonyldiimidazole der allgemeinen Formeln Ia, Ib, Ic oder Gemische davon sind durch Umsetzung mindestens eines Imidazols der allgemeinen Formeln IIa und IIb mit Phosgen in einem substituierten aromatischen Kohlenwasserstoff als Lösungsmittel zugänglich, wobei das weiterhin gebildete Hydrochlorid des mindestens einen Imidazols der allgemeinen Formeln IIa und IIb als Schmelze durch Phasentrennung aus dem Reaktionsgemisch entfernt wird.

Die Herstellung von Carbonyldiimidazol aus Imidazol und Phosgen ist allgemein aus EP-A-0 692 476 bekannt. Bei der Umsetzung (siehe Beispiel 1) fällt jedoch Imidazolhydrochlorid als Feststoff an, der abfiltriert werden muß. Dazu ist ein Umfüllen des Reaktionsgemisches erforderlich, das nachteilig ist, da das gebildete Carbonyldiimidazol sehr feuchtigkeitsempfindlich ist und die Aufarbeitung deshalb erschwert ist.

Erfindungsgemäß wurde gefunden, daß die Hydrochloride der Imidazole der allgemeinen Formeln IIa und IIb unter den Reaktionsbedingungen als Flüssigkeiten (Schmelze) anfallen und deshalb durch eine einfache Phasentrennung aus dem Produktgemisch praktisch vollständig abgetrennt werden können. Die so erhaltenen Hydrochloride können durch Neutralisation wieder in die Imidazole der allgemeinen Formeln IIa und IIb überführt und in die Synthese zurückgeführt werden.

Gegenüber dem in EP-A-0 692 476 beschriebenen Verfahren ist die Abtrennung des Hydrochlorids somit wesentlich vereinfacht und das Risiko einer Zersetzung der gewünschten Carbonyldiimidazole vermindert.

Die Carbonyldiimidazole der allgemeinen Formeln Ia, Ib und Ic werden bei der Umsetzung von Imidazolen der allgemeinen Formeln IIa und/oder IIb als Gemisch gebildet. Es kann eine Verschiebung der Doppelbindungen und Protonen in den Imidazolen bei der Umsetzung auftreten, so daß auch aus einer einzelnen Verbindung der allgemeinen Formeln IIa und IIb ein Produktgemisch erhalten wird. Dabei wird jedoch überwiegend das Produkt erhalten, bei dem keine Verschiebung von Doppelbindung und Proton notwendig ist. Die Carbonyldiimidazole der allgemeinen Formeln Ia, Ib, Ic oder Gemische davon können durch Abkühlen des Reaktionsgemisches mittels Ausfällen isoliert werden. Sie können jedoch auch nach Abtrennung der Hydrochloride der Imidazole direkt im Reaktionsgemisch weiterverarbeitet werden, ohne daß eine Isolierung erforderlich ist. Dabei kann die heiße Rohlösung ohne weitere Aufarbeitung eingesetzt werden.

Die Carbonyldiimidazole der allgemeinen Formeln Ia, Ib, Ic oder Gemische davon können für eine Vielzahl von Umsetzungen eingesetzt werden, beispielsweise zur Herstellung von entsprechenden Imidazol-1-carbonsäure-tert.-butylestern, zur Carbonylgruppenübertragung, oder zur Dehydratisierung, insbesondere bei der Herstellung von Estern und Amiden aus Carbonsäuren.

In der Regel legt man das mindestens eine Imidazol der allgemeinen Formeln IIa und IIb im Lösungsmittel vor und leitet bei einer Temperatur im Bereich von 60 bis 130°C, vorzugsweise 80 bis 130°C, insbesondere 90 bis 130°C Phosgen ein. Als Lösungsmittel sind alle dabei geeigneten substituierten aromatischen Kohlenwasserstoffe einsetzbar. Beispiele vorzugsweise eingesetzter Lösungsmittel sind Chlorbenzol, Xylol und o-Dichlorbenzol. Nicht umgesetztes Phosgen kann durch Strippen mit Inertgasen wie Stickstoff entfernt werden. Das erhaltene Reaktionsgemisch kann gegebenenfalls weiter erhitzt werden, bis sich ein flüssiges Zweiphasensystem ausbildet und eine Phasentrennung möglich wird.

Die Umsetzung der Carbonyldiimidazole der allgemeinen Formeln Ia, Ib, Ic oder Gemische davon zu den Imidazolcarbonsäureestern der allgemeinen Formeln IIIa und IIIb oder Gemischen davon erfolgt durch Umsetzung mit tert.-Butanol in einem substituierten aromatischen Kohlenwasserstoff als Lösungsmittel. Dabei wird vorzugsweise ein wie vorstehend definiertes Lösungsmittel eingesetzt, das insbesondere mit dem im ersten Reaktionsschritt verwendeten Lösungsmittel identisch ist. Das im ersten Reaktionsschritt erhaltene Reaktionsgemisch kann somit ohne Abtrennung des Lösungsmittels weiter verwendet werden. Die Umsetzung mit tert.-Butanol erfolgt vorzugsweise bei Temperaturen im Bereich von 50 bis 140°C, besonders bevorzugt 60 bis 120°C, insbesondere 70 bis 90°C. Bei einer niedrigeren Temperatur läßt sich dabei die Ausbeute erhöhen. Bei der Umsetzung von Carbonyldiimidazolen der allgemeinen Formeln Ia, Ib, Ic oder Gemischen davon mit tert.-Butanol werden neben den gewünschten tert.-Butylestern auch Imidazole der allgemeinen Formeln IIa und IIb gebildet. Diese Imidazole können durch Extrahieren des erhaltenen Reaktionsgemisches mit Wasser abgetrennt werden. Aus der wäßrigen Imidazolphase können sie dann isoliert und in das Herstellungsverfahren der Carbonyldiimidazole zurückgeführt werden.

Es wurde erfindungsgemäß gefunden, daß das erhaltene Reaktionsgemisch mit Wasser extrahiert werden kann, ohne daß sich die Imidazolcarbonsäureester der allgemeinen Formeln IIIa, IIIb oder Gemische davon zersetzen (hydrolysieren). Daher ist eine einfache Extraktion mit Wasser möglich, wobei die gewünschten Imidazolcarbonsäureester in der organischen Phase erhalten werden. Sie können nach Abdestillieren des Lösungsmittels unter vermindertem Druck fraktioniert destilliert werden, um zu den reinen Imidazolcarbonsäureestern der allgemeinen Formeln IIIa, IIIb oder Gemischen davon zu gelangen.

Die Erfindung betrifft auch ein Verfahren zur Abtrennung von Imidazolen aus Reaktionsgemischen, die Imidazole der allgemeinen Formeln IIa und IIb oder Gemische davon und Imidazolcarbonsäureester der allgemeinen Formeln IIIa und IIIb oder Gemische davon enthalten, durch Extrahieren des Reaktionsgemisches mit Wasser.

Die Imidazolcarbonsäureester der allgemeinen Formeln IIIa, IIIb oder Gemische davon können auch durch Umsetzung mindestens eines Imidazols der allgemeinen Formeln IIa und IIb mit Pyrokohlensäuredi-tert.-butylester (BOC-Anhydrid) hergestellt werden. Dabei entstehen als Nebenprodukte Kohlendioxid und tert.-Butanol. Wegen der hohen Kosten von BOC-Anhydrid ist erfindungsgemäß jedoch das zuvor beschriebene Verfahren bevorzugt.

In den Verbindungen der vorstehenden Formeln sind R¹ ein C₁₋₄-Alkylrest und R² Wasserstoff oder ein Methylrest. Vorzugsweise ist R¹ ein C₁₋₄-Alkylrest und R² Wasserstoff. R¹ kann dabei ein Methyl-, Ethyl-, n-Propyl-, i-Propyl-, n-Butyl-, i-Butyl-, oder tert.-Butylrest sein. Bevorzugt ist R¹ ein C₁₋₃-Alkylrest, insbesondere ein Methyl- oder Ethylrest, speziell ein Methylrest.

Die speziell bevorzugten Carbonyldiimidazole der allgemeinen Formeln Ia, Ib, Ic sind somit Carbonyldi-4-methylimidazol, Carbonyl-4-methylimidazol-5-methylimidazol und Carbonyldi-5-methylimidazol. Die eingesetzten Imidazole der allgemeinen Formeln IIa und IIb sind dabei 4-Methylimidazol und 5-Methylimidazol. Besonders bevorzugt wird 4-Methylimidazol bei der Synthese eingesetzt. Dabei wird überwiegend Carbonyldi-4-methylimidazol erhalten.

Bei der Umsetzung der isomeren Carbonyldimethylirnidazole mit tert.-Butanol werden als Imidazolcarbonsäureester der allgemeinen Formeln IIIa, IIIb 5- und 4-Methylimidazol-l-carbonsäure-tert.-butylester erhalten. Bei ursprünglich ausschließlicher Verwendung von 4-Methylimidazol wird überwiegend das 4-Methylimidazolisomere erhalten. Der Anteil des 5-Methylimidazolisomeren beträgt etwa 6 bis 7 %. Das Produktgemisch kann bei einem Siedepunkt von etwa 63 bis 64°C bei 0,4 bar unzersetzt destilliert werden.

Die Imidazolcarbonsäureester der allgemeinen Formeln IIIa und IIIb oder Gemische davon können zum Einführen von BOC-Schutzgruppen in Aminofunktionen verwendet werden. Ein allgemeines Verfahren dafür ist in Spec. Chem. 13 (1993), 67 bis 69 beschrieben. Dabei werden Aminofunktionen enthaltende Verbindungen mit Imidazolcarbonsäureestern der allgemeinen Formeln IIIa und IIIb oder Gemischen davon umgesetzt. Dabei werden die Imidazole wieder freigesetzt, so daß einheitliche Schutzgruppen erhalten werden, unabhängig vom eingesetzten Isomeren des Carbonsäureesters.

Nach dem erfindungsgemäßen Verfahren können die Imidazolcarbonsäureester der allgemeinen Formeln IIIa, IIIb und Gemische davon, die bei Raumtemperatur flüssig sind und einfach destilliert werden können, in einem Eintopfverfahren ohne Aufarbeitung des Zwischenproduktes aus kostengünstigen, leicht zugänglichen Chemikalien hergestellt werden.

Die Erfindung wird nachstehend anhand von Beispielen näher erläutert.

### Beispiele

### Beispiel 1

### Herstellung von Carbonyl-di-4-methylimidazol:

2 Mol 4-Methylimidazol wurden in Chlorbenzol, welches durch Andestillieren entwässert wurde, gelöst. Danach wurden bei 60°C 0,5 mol Phosgen eingeleitet (bis Phosgenrückfluß). Nach vollständiger Reaktion ließ man 1 h nachreagieren, danach wurde das überschüssige Phosgen mit Stickstoff ausgestrippt. Die gebildete Suspension wurde auf 80 bis 100°C erhitzt, bis die untere Phase vollständig geschmolzen war. Die untere Phase wurde dann vollständig abgetrennt. Sie enthielt ausschließlich Methylimidazol-hydrochlorid.

Die obere Phase enthielt das Carbonyl-di-4-methylimidazol, welches beim Abkühlen unvollständig auskristallisierte. Carbonyl-di-4-methylimidazol kann entweder durch Eindampfen des Lösungsmittels unter vermindertem Druck isoliert oder direkt in Lösung weiterverarbeitet werden. Das Carbonyl-di-4-methylimidazol enthält immer auch einen Anteil an isomerem Carbonyl-4-methylimidazol-5-Methylimidazol, welches durch Phosgenierung am anderen N-Atom entsteht. Für die Weiterverwendung des Produktes ist dies unwichtig, da beim Schutz der entsprechenden Aminosäure kein Imidazolrest mehr im Zielmolekül verbleibt.

| | | | | | | |
|---|---|---|---|---|---|---|
| Schmelzpunkt des | | | | | | |
| Carbonyl-di-4-methylimidazols: | 101 bis 107°C | | | | | |
| Ausbeute nach Isolation: | 89 % | | | | | |
| IR: | Carbonylbande bei 1717 cm⁻¹ | | | | | |
| Berechnet für C₉H₁₀N₄O: | C: | 56,83 | H: | 5,30 | N: | 29,46 |
| Gefunden: | C: | 56,50 | H: | 5,30 | N: | 29,60 |

### Beispiel 2

### Herstellung von 4-Methylimidazol-1-carbonsäure-tert.-butylester:

2 Mol 4-Methylimidazol wurden in Xylol, welches durch Andestillieren entwässert wurde, gelöst. Danach wurden bei 60°C 0,5 mol Phosgen eingeleitet (bis Phosgenrückfluß). Nach vollständiger Reaktion ließ man 1 h nachreagieren, danach wurde das überschüssige Phosgen mit Stickstoff ausgestrippt. Die gebildete Suspension wurde auf 130°C erhitzt, bis die untere Phase vollständig geschmolzen war. Die untere Phase wurde vollständig abgetrennt.

Die Xylollösung des Carbonyl-di-4-methylimidazols (obere Phase) wurde mit einer äquimolaren Menge an tert.-Butanol versetzt und zwei Stunden unter Rückfluß gekocht. In der GC-Kontrolle wurde der Umsatz überprüft. (Bildung von 4-Methyl-imidazol und 4-Methylimidazol-l-carbonsäure-tert.-butylester, Verschwinden der tert.-Butanol- und der Carbonyl-di-4-methylimidazol-Bande).

Nach vollständigem Umsatz wurde die Reaktionslösung zweimal mit Wasser extrahiert, um das 4-Methyl-imidazol aus der Lösung zu entfernen.

Die organische Phase wurde fraktioniert destilliert. Das Produkt (tert.-Butyloxicarbonyl-4-methylimidazol) ging bei 63 bis 64°C und 0,4 mbar über.

| | | | | | | |
|---|---|---|---|---|---|---|
| IR: | Carbonylbande bei 1752 cm⁻¹ | | | | | |
| Berechnet für C₉H₁₄N₂O₂: | C: | 59,32 | H: | 7,74 | N: | 15,37 |
| Gefunden: | C: | 59,10 | H: | 7,60 | N: | 15,50 |

Das Produkt enthält einen Anteil an isomerem 5-Methylimidazol-1-carbonsäure-tert.-butylester (6 bis 7 %). Für die Anwendung als Schutzgruppenreagenz ist dies ohne Bedeutung, da ausschließlich die BOC-Gruppe übertragen wird.

### Beispiel 3:

### Alternativer Syntheseweg

Zu einer Lösung von 16,4 g (0,2 Mol) 4-Methylimidazol und 1,2 g (0,01 Mol) 4-Dimethylaminopyridin (DMAP) in 250 ml trockenem Acetonitril wurden unter N₂ und unter Rühren bei 18 °C 47,96 g (0,22 Mol) Pyrokohlensäure-di-tert.-butylester (BOC-Anhydrid) während ca. 1 Minute hinzugegeben. Die Reaktionslösung erwärmte sich innerhalb von 5 Minuten auf 22°C und man rührte 1 Stunde nach. Nach dem Einengen am Rotationsverdampfer bei 10 bis 20 mbar und 45°C verblieben 37,5 g flüssiger Rückstand, der im Vakuum (1 mbar) kurzwegdestilliert wurde.

Bei einer Sumpftemperatur von 82°C und einer Kopftemperatur von 65°C wurden 28,0 g Destillationshauptlauf als klare, leicht bewegliche Flüssigkeit erhalten, die nach GC- und NMR-Analyse wie folgt zusammengesetzt war:

| | |
|---|---|
| 83,0 % | 4-Methylimidazol-1-carbonsäure-tert.-butylester, |
| 11,0 % | 5-Methylimidazol-1-carbonsäure-tert.-butylester |

Im Destillationsvorlauf (6,5 g) waren weitere 5,4 g 4-Methylimidazol-1-carbonsäure-tert.-butylester sowie 0,5 g 5-Methylimidazol-1-carbonsäure-tert.-butylester enthalten. (Destillationsrückstand: 0,7 g).

Die Gesamtausbeute an 4-Methylimidazol-1-carbonsäure-tert.-butylester betrug damit 32,2 g (0,18 Mol), 88 %.

### Beispiel 4

### Verbessertes Verfahren

1020 g Chlorbenzol wurden durch Abdestillieren von 20 g Chlorbenzol entwässert. 164 g 4-Methylimidazol (2 Mol) wurden in der Schmelze zugegeben und gelöst. Bei 60 bis 70°C Innentemperatur wurden innerhalb von 30 Minuten 50 g (0,5 mol) Phosgen eingegast. Nach einer Stunde Nachreaktion wurde mit Stickstoff gestrippt; dabei wurde solange erhitzt, bis die feste untere Phase vollständig geschmolzen war (Innentemperatur 95°C). Die untere Phase wurde dann bei 95°C abgetrennt (156 g).

Zur Chlorbenzolphase wurden innerhalb von 10 Minuten bei 82 bis 76°C 74 g (1 Mol) tert.-Butanol zugetropft und die Reaktionslösung 5 h lang bei 80°C gerührt. Der Austrag wurde zweimal mit je 100 ml Wasser extrahiert. Nach dem Abdestillieren des Lösungsmittels im Vakuum wurde der Rückstand (85 g) fraktioniert destilliert. Die Hauptfraktion ging bei 1,5 mbar und 78 bis 83°C über. Es wurden 66,2 g 4-Methylimidazol-1-carbonsäure-tert.-butylester destilliert, das entspricht einer Ausbeute von 72,7 %. Das Produkt enthielt ca. 4 % des 5-Methylimidazol-Isomeren.

### Beispiel 5

### Einführung der BOC-Schutzgruppe auf die Aminogruppe von D,L-Alanin

8,9 g D,L-Alanin (0,1 Mol), 75 g DMF und 30,4 g Diaza-bicycloundecen ("DBU", 0,2 Mol) werden vorgelegt. Innerhalb von 10 Minuten werden 23 g (0,12 mol) 4-Methylimidazol-1-earbonsäure-tert.-butylester so zugetropft, daß die Temperatur nicht über 5°C steigt. Danach wird 20 h bei Raumtemperatur gerührt. Der Austrag wird bei 0 bis 5°C mit 10%iger HCl auf pH 2,5 eingestellt. Es wird einmal mit Methyl-tert.-butylether (MTBE) extrahiert. Die organische Phase wird im Vakuum eingeengt, wobei der Rückstand teilweise kristallisiert. Nach Aufschlämmen mit Cyclohexan werden farblose Kristalle vom Smp. 105 bis 106°C erhalten. NMR-Spektrum und Elementaranalyse stimmen mit Referenzdaten überein.

## Patentansprüche

1. Carbonyldiimidazole der allgemeinen Formeln Ia, Ib, Ic oder Gemische davon 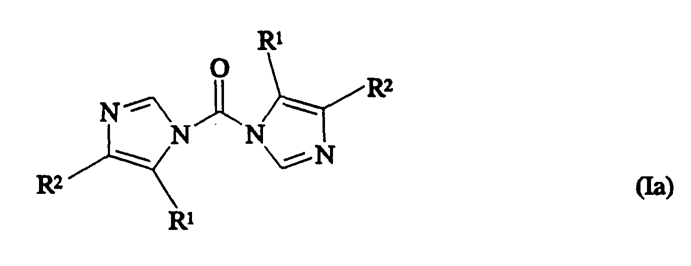 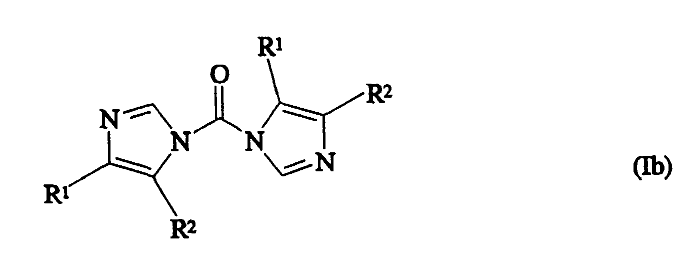 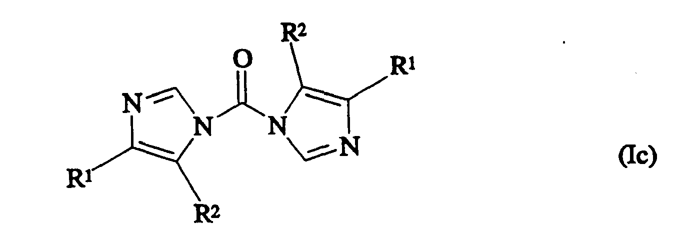 wobei R¹ ein C₁₋₄-Alkylrest und R² Wasserstoff oder ein Methylrest ist.

2. Verfahren zur Herstellung von Carbonyldiimidazolen der allgemeinen Formeln Ia, Ib, Ic oder Gemischen davon nach Anspruch 1 durch Umsetzung mindestens eines Imidazols der allgemeinen Formeln IIa und IIb 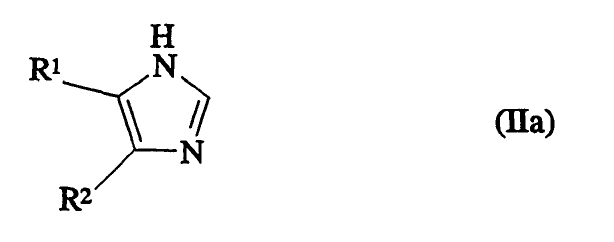 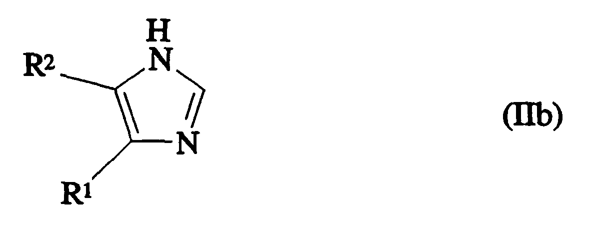 in denen R¹ und R² die in Anspruch 1 angegebene Bedeutung haben,
mit Phosgen in einem substituierten aromatischen Kohlenwasserstoff als Lösungsmittel,
wobei das weiterhin gebildete Hydrochlorid des mindestens einen Imidazols der allgemeinen Formeln IIa und IIb als Schmelze durch Phasentrennung aus dem Reaktionsgemisch entfernt wird.

3. Verwendung von Carbonyldiimidazolen der allgemeinen Formeln Ia, Ib, Ic oder Gemischen davon nach Anspruch 1 zur Herstellung von entsprechenden Imidazol-1-carbonsäure-tert.-butylestern, zur Carbonylgruppenübertragung oder zur Dehydratisierung.

4. Imidazolcarbonsäureester der allgemeinen Formeln IIIa und IIIb oder Gemische davon 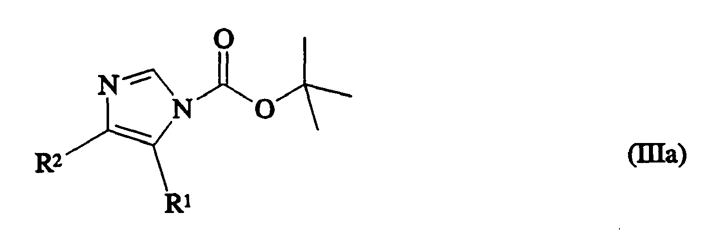 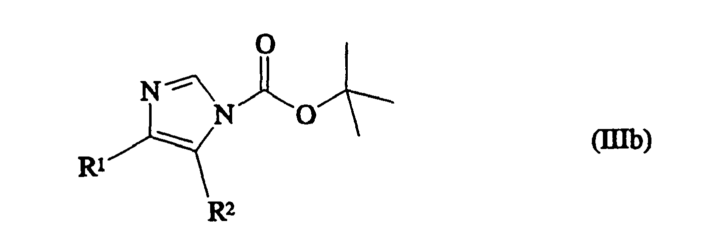 wobei R¹ ein C₁₋₄-Alkylrest und R² Wasserstoff oder ein Methylrest ist.

5. Verfahren zur Herstellung von Imidazolcarbonsäureestern der allgemeinen Formeln IIIa, IIIb oder Gemischen davon nach Anspruch 4 durch Umsetzung von Carbonyldiimidazolen der allgemeinen Formeln Ia, Ib, Ic oder Gemischen davon nach Anspruch 1 mit tert.-Butanol in einem substituierten aromatischen Kohlenwasserstoff als Lösungsmittel und Extrahieren des erhaltenen Reaktionsgemisches mit Wasser zur Entfernung von weiterhin gebildeten Imidazolen der allgemeinen Formeln IIa und IIb, wie sie in Anspruch 2 definiert sind.

6. Verfahren zur Abtrennung von Imidazolen aus Reaktionsgemischen, die Imidazole der allgemeinen Formeln IIa und IIb oder Gemische davon, wie sie in Anspruch 2 definiert sind, und Imidazolcarbonsäureester der allgemeinen Formeln IIIa und IIIb oder Gemische davon, wie sie in Anspruch 4 definiert sind, enthalten, durch Extrahieren des Reaktionsgemisches mit Wasser.

7. Verfahren zur Herstellung von Imidazolcarbonsäureestern der allgemeinen Formeln IIIa, IIIb, oder Gemischen davon nach Anspruch 4 durch Umsetzung mindestens eines Imidazols der allgemeinen Formeln IIa und IIb, wie sie in Anspruch 2 definiert sind, mit Pyrokohlensäuredi-tert.-butylester.

8. Verwendung von Imidazolcarbonsäureestern der allgemeinen Formeln IIIa und IIIb oder Gemischen davon, wie sie in Anspruch 4 definiert sind, zum Einführen von BOC-Schutzgruppen in Aminofunktionen.

9. Verfahren zum Einführen von BOC-Schutzgruppen in Aminofunktionen durch Umsetzung von Aminofunktionen enthaltenden Verbindungen mit Imidazolcarbonsäureestern der allgemeinen Formel IIIa und IIIb oder Gemischen davon, wie sie in Anspruch 4 definiert sind.

10. Verbindungen, Verfahren oder Verwendungen nach einem der Ansprüche 1 bis 9, **dadurch gekennzeichnet, daß** R¹ ein Methylrest und R² Wasserstoff ist.

## Claims

1. A carbonyldiimidazole of the formula Ia, Ib, Ic or mixture thereof 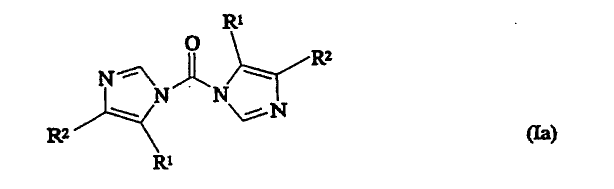 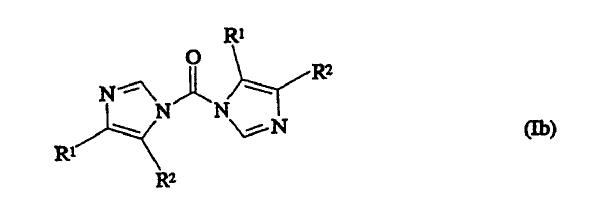 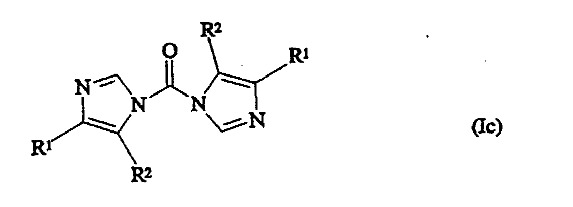 where R¹ is C₁₋₄-alkyl and R² is hydrogen or methyl.

2. A process for preparing a carbonyldiimidazole of the formula Ia, Ib, Ic or mixture thereof according to claim 1 by reacting at least one imidazole of the formula IIa or IIb 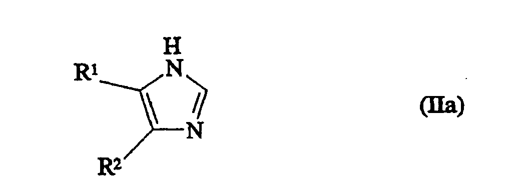 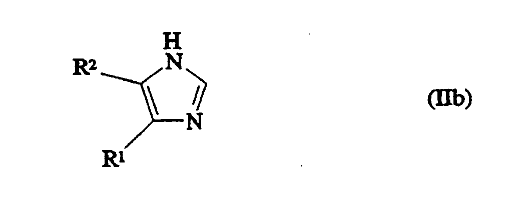 where R¹ and R² have the meanings indicated in claim 1,
with phosgene in a substituted aromatic hydrocarbon as solvent,
wherein the hydrochloride, which is also formed, of the at least one imidazole of the formula IIa or IIb is removed from the reaction mixture by phase separation as melt.

3. The use of carbonyldiimidazole of the formula Ia, Ib, Ic or mixture thereof according to claim 1 for preparing corresponding tert-butyl imidazole-1-carboxylates, for carbonyl group transfer, or for dehydration.

4. An imidazolecarboxylic ester of the formula IIIa or IIIb or mixture thereof 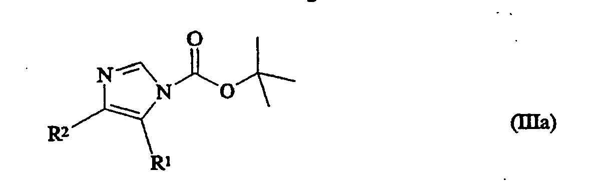 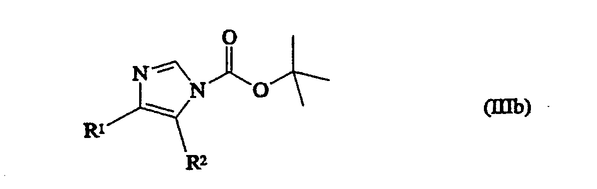 where R¹ is C₁₋₄-alkyl and R² is hydrogen or methyl.

5. A process for preparing an imidazolecarboxylic ester of the formula IIIa, IIIb or mixture thereof according to claim 4 by reacting a carbonyldiimidazole of the formula Ia, Ib, Ic or mixture thereof according to claim 1 with tert-butanol in a substituted aromatic hydrocarbon as solvent and extracting the resulting reaction mixture with water to remove imidazoles, which are also formed, of the formulae IIa and IIb as defined in claim 2.

6. A process for removing imidazoles from reaction mixtures which comprise imidazoles of the formulae IIa and IIb or mixtures thereof as defined in claim 2, and imidazolecarboxylic esters of the formulae IIIa and IIIb or mixtures thereof as defined in claim 4, by extracting the reaction mixture with water.

7. A process for preparing an imidazolecarboxylic ester of the formula IIIa, IIIb or mixture thereof according to claim 4 by reacting at least one imidazole of the formula IIa or IIb as defined in claim 2 with di-tert-butyl pyrocarbonate.

8. The use of an imidazolecarboxylic ester of the formula IIIa or IIIb or mixture thereof as defined in claim 4 for introducting BOC protective groups into amino functionalities.

9. A process for introducing BOC protective groups into amino functionalities by reacting compounds con-taining amino functionalities with imidazolecarboxylic esters of the formulae IIIa and IIIb or mixtures thereof as defined in claim 4.

10. A compound, process or use according to any of claims 1 to 9, wherein R¹ is methyl and R² is hydrogen.

## Revendications

1. Carbonyldiimidazoles de formules générales Ia, Ib, Ic ou mélanges de ceux-ci 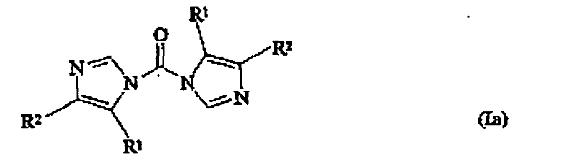 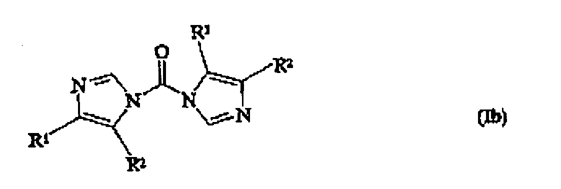 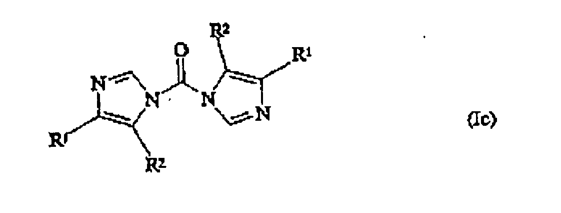 dans lesquelles R¹ est un radical alkyle en C₁₋₄ et R² est hydrogène ou un radical méthyle.

2. Procédé de fabrication de carbonyldiimidazoles de formules générales Ia, Ib, Ic ou des mélanges de ceux-ci selon la revendication 1 par transformation d'au moins un imidazole de formules générales IIa et IIb 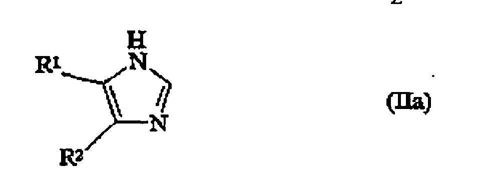 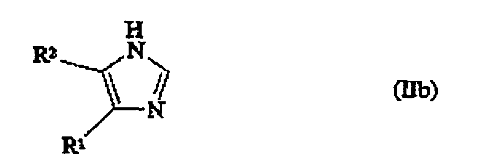 dans lesquelles R¹ et R² ont la signification donnée à la revendication 1,
avec comme solvant du phosgène dans un hydrocarbure aromatique substitué,
l'hydrochlorure formé en outre de l'au moins un imidazole de formules générales IIa et IIb étant éliminé du mélange réactionnel sous forme de fusion par séparation des phases.

3. Utilisation de carbonyldiimidazoles de formules générales Ia, Ib, Ic ou des mélanges de ceux-ci selon la revendication 1 pour la fabrication de t-butylesters d'acide imidazol-1-carboxylique pour le transfert de groupes carbonyle ou pour la déshydratation.

4. Esters d'acide imidazolcarboxylique de formules générales IIIa et IIIb ou mélanges de ceux-ci 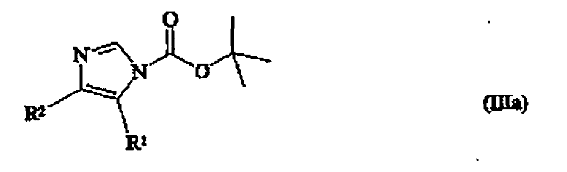 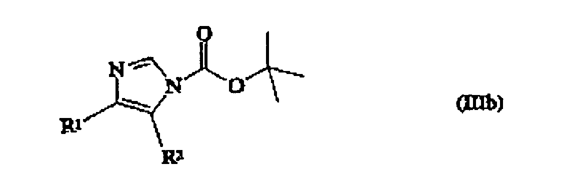 dans lesquelles R¹ est un radical alkyle en C₁₋₄ et R² est l'hydrogène ou un radical méthyle.

5. Procédé de fabrication d'esters d'acide imidazolcarboxylique de formules générales IIIa, IIIb ou des mélanges de ceux-ci selon la revendication 4 par réaction de carbonyldiimidazoles de formules générales Ia, Ib, Ic ou des mélanges de ceux-ci selon la revendication 1 avec du t-butanol dans un hydrocarbure aromatique substitué comme solvant et extraction du mélange réactionnel obtenu avec de l'eau pour l'élimination des imidazoles formés en outre des formules générales IIa et IIb, telles qu'ils sont définis à la revendication 2.

6. Procédé de séparation d'imidazoles de mélanges réactionnels, qui contiennent des imidazoles de formules générales IIa et IIb ou des mélanges de ceux-ci, tels qu'ils sont définis à la revendication 2 et des esters d'acide imidazolcarboxylique de formules générales IIIa et IIIb ou des mélanges de ceux-ci, tels qu'ils sont définis à la revendication 4, par extraction du mélange réactionnel avec de l'eau.

7. Procédé de fabrication d'esters d'acide imidazolcarboxylique de formules générales IIIa, IIIb ou des mélanges de ceux-ci selon la revendication 4 par réaction d'au moins un imidazole de formules générales IIa et IIb, tels qu'ils sont définis à la revendication 2, avec des t-butylesters d'acide pyrocarbonique.

8. Utilisation d'esters d'acide imidazolcarboxylique de formules générales IIIa et IIIb ou des mélanges de ceux-ci, tels qu'ils sont définis à la revendication 4, pour l'introduction de groupes protecteurs BOC en fonctions amino.

9. Procédé d'introduction de groupes protecteurs BOC en fonctions amino par réaction de composés contenant des fonctions amino avec des esters d'acide imidazolcarboxylique de formules générales IIIa et IIIb ou des mélanges de ceux-ci, tels qu'ils sont définis à la revendication 4.

10. Composés, procédés ou utilisations selon l'une des revendications 1 à 9, **caractérisés en ce que** R¹ est un radical méthyle et R² est un hydrogène.
